# EUROPEAN PATENT APPLICATION

(11) **EP 2 701 089 A2**
(43) Date of publication of application: **26.02.2014**
(21) Application number: 13180982.4
(22) Date of filing: 20.08.2013
(51) Int. Cl.: G06F 19/00

(54) **Patient monitoring device**

(30) Priority: 23.08.2012 SE 1200508
(71) Applicant: CareLigo AB, 184 44 Akersberga (SE)
(72) Inventor: Blomqvist, Andreas, SE-187 76 Täby (SE); Westman, Fredrik, SE-184 44 Åkersberga (SE)
(74) Representative: Sjölander, Henrik

(57) **Abstract**

A patient monitoring device (100, 200, 300) receives sensor data from an external sensor (400). The sensor data is processed by a drug titration module (120, 220, 320) to automatically provide information of current dose a prescribed drug calculated according to a drug titration algorithm. An education module (140, 240, 340) uses the sensor data and user preferences to select an education class among multiple available education classes and provides an education instruction belonging to the selected education class. This provided education instruction is display with the information of the current dose on a screen (150, 250, 350).

## Description

### TECHNICAL FIELD

The present invention generally relates to medical devices, and in particular to patient monitoring devices and to systems incorporating such devices.

### BACKGROUND

Heart failure is a very common disease in industrial countries. In Sweden alone there are about 250 000 people suffering from heart failure. The prevalence is 2-3 % among the whole population and as high as 10-15 % among those above 75 years. The prognosis for heart failure patients is in fact worse than for patients diagnosed with breast or prostate cancer, with a five year mortality of about 50 %.

Heart failure is intimately associated with high healthcare costs. Today about 2-3 % of the total healthcare budget in Sweden is spent on treatment of heart failure. However, in clear contrast to other medical conditions associated with high healthcare costs, the medicament related cost constitutes merely about 2-8 % of the total cost for heart failure treatment, whereas costs associated with hospitalization amounts to about 70 %.

During the last decade more than 30 randomized clinical studies with thousand of patients have been conducted with the purpose of studying various ways of reducing the frequency of hospitalization of heart failure patients. These studies demonstrate that different intervention modalities, such as remote monitoring of physiological parameters, patient education, house calls and telephone support and follow-up, could reduce the frequency of hospitalization to about half as compared to control groups. These various intervention modalities had the effect of increasing the patients' compliance to the prescribed treatment, and it is widely agreed upon that increasing patient compliance (or adherence) is the most impactful way to reduce heart failure associated costs

Similar problems with regard to lack of compliance to prescribed treatment and high hospitalization costs are also found among other elderly patient groups than heart failure patients. Various techniques have been proposed to solve these problems.

US 2009/0062727 discloses a system for controlling body fluids by automatically infusing diuretic and/or other drugs into a human patient. The rate of infusion of the diuretic is adjusted based on the measured weight of the patient. This weight is transmitted wirelessly to a portable diuretic infusion device attached to the patient.

US 2010/0228566 discloses a portable vital statistics monitoring and medication dispensing system that provides personal healthcare management and daily prescription routines. The system incorporates a medical dispenser, a blood testing system and other vital sign monitoring into a portable wireless device. The system notifies the user of the correct times to take medication and automatically dispenses the accurate prescription dosage.

US 2007/0260487 discloses a system for managing a prescribed drug regimen for a user with an electronic device for interactive communication with the user and a server. The server includes an analysis unit having access to a database of drug characteristics and a processor for comparing sensed characteristics of a drug in an electronic device so as to identify the drug and provide, to the electronic device, an indication of compatibility of the drug with the drug regimen of the patient.

Suh et al., WANDA B.: Weight and Activity with Blood Pressure Monitoring System for Heart Failure Patients, IEEE Trans Inf Technol Biomed, 2010 June 14: 1-6 discloses WANDA B. wireless health technology that leverages sensor technology and wireless communication to monitor heart failure patient activity and to provide tailored guidance. Patients who have cardiovascular system disorders can measure their weight, blood pressure, activity levels, and other vital signs in real-time automated fashion.

US 2011/0082711 discloses a person health organizer receiving physiological reading data from signals acquired by a sensor. The personal health organizer integrates reading data with applications that assist and promote health management, including reminders, alerts and health data tracking.

US 2011/0124996 discloses diabetes health management systems for use in portable devices. A data module of the system receives and stores blood glucose measurement values, insulin dosage data and health data entries. A therapy module determines a therapy advice message based on the received blood glucose measurements values and the received insulin dosage data and displays the therapy advise message on a user interface.

US 2012/0108984 discloses an integrated patient care system that creates and automatically distribute a therapy regimen to a patient. A clinician module of the system is used to create thereapy instructions with one or more therapy regimens. A therapy module of the system may use the therapy instructions to automatically select a therapy regimen based on a patient condition detected based on a sensed physiological parameter.

The above described systems and devices are, however, all marred by being complex or hard to operate for the patients. Hence, there is a need for an improvement within this technical field.

### SUMMARY

It is a general objective to provide a patient monitoring device that can be used as a tool by patients for treatment compliance.

It is a particular objective to provide such a tool that may reduce the risk for hospitalization of the patients.

These and other objectives are met by embodiments as disclosed herein.

An aspect of the embodiments relates to a patient monitoring device comprising a communication unit configured to receive, from an external sensor, a signal representative of a current value of a medical characteristic of a patient. A memory is connected to the communication unit and configured to store the current value. A drug titration module is connected to the memory and is configured to process the current value according to a drug titration algorithm stored in the memory. The drug titration module thereby outputs information of a current dose of a prescribed drug. A screen is configured to present, to the patient, information of multiple predefined education classes. Each such education class comprises at least one respective education instruction. The patient monitoring device also comprises a user input configured to generate a class selection signal representative of an education class selected by the patient by activating the user input. The memory is then configured to store, based on the selection signal, information of the education class selected by the patient. An education module is implemented connected to the memory. The education module comprises a class selector configured to select, at least partly based on the current value and the information of the education class selected by the patient, an education class among the multiple predefined education class. An education instruction provider of the education module is configured to provide, from the memory, an education instruction belonging to the education class selected by the class selector. The screen of the patient monitoring device is configured to present information of the current dose and the education instruction provided by the education instruction provider.

Another aspect of the embodiments relates to a heart failure monitoring system comprising a weight scale with a processor configured to generate a signal representative of a current weight of a patient standing on the weight scale. The weight scale also comprises a communication unit configured to transmit the signal to a communication unit of a patient monitoring device. The heart failure monitoring system also comprises a screen configured to present, to the patient, information of multiple predefined education classes. Each such education class comprises at least one education instruction. The heart failure monitoring system further comprises a patient monitoring device according to above. In this another aspect, the drug titration module is configured to process the value according to a drug titration algorithm, stored in the memory, to output information of a current dose of a prescribed diuretic drug.

The embodiments provide a valuable tool to help the patient with his/her treatment and are designed to promote treatment compliance and adherence. As a consequence, the risk of hospitalization of the patient may be reduced by a more correct treatment compliance. The embodiments thereby add to the patient's quality of life by putting them at the center, and in the control of their treatment.

### BRIEF DESCRIPTION OF THE DRAWINGS

The invention, together with further objects and advantages thereof, may best be understood by making reference to the following description taken together with the accompanying drawings, in which:
Fig. 1 is a schematic overview of a patient monitoring system comprising an external sensor and a patient monitoring device according to an embodiment;
Fig. 2 is a schematic block diagram of an education module according to an embodiment;
Fig. 3 is a schematic block diagram of a drug titration module according to an embodiment;
Fig. 4 is a schematic block diagram of a patient monitoring device according to another embodiment;
Fig. 5 is a schematic block diagram of a patient interaction module according to an embodiment;
Fig. 6 is a schematic block diagram of a patient monitoring device according to a further embodiment;
Fig. 7 illustrates an embodiment of a patient monitoring device;
Fig. 8 illustrates presentation of information of current dose together with a provided education instruction according to an embodiment;
Fig. 9 illustrates presentation of available education classes according to an embodiment;
Fig. 10 illustrates presentation of patient data available using a patient interaction module according to an embodiment;
Fig. 11 illustrates presentation of contact information to healthcare facility according to an embodiment;
   and
Fig. 12 illustrates an embodiment connecting a patient monitoring system with external devices and services.

### DETAILED DESCRIPTION

Throughout the drawings, the same reference numbers are used for similar or corresponding elements.

The present embodiments generally relate to a patient monitoring device and a patient monitoring system incorporating such a device. The patient monitoring device can be used as an intuitive tool to increase a patient's compliance to a prescribed drug regime and/or treatment instructions from the patient's physician. The patient monitoring device is in particular suitable for usage in connection with elderly patients or other patient groups where there is a risk that the patient may have low compliance or adherence to a prescribed treatment or find it hard to interpret a prescribed treatment.

The present embodiments lower the thresholds for the patient to adhere to a prescribed treatment and regimen and can therefore be a significant tool and help to prevent hospitalization and deterioration of the patient's health.

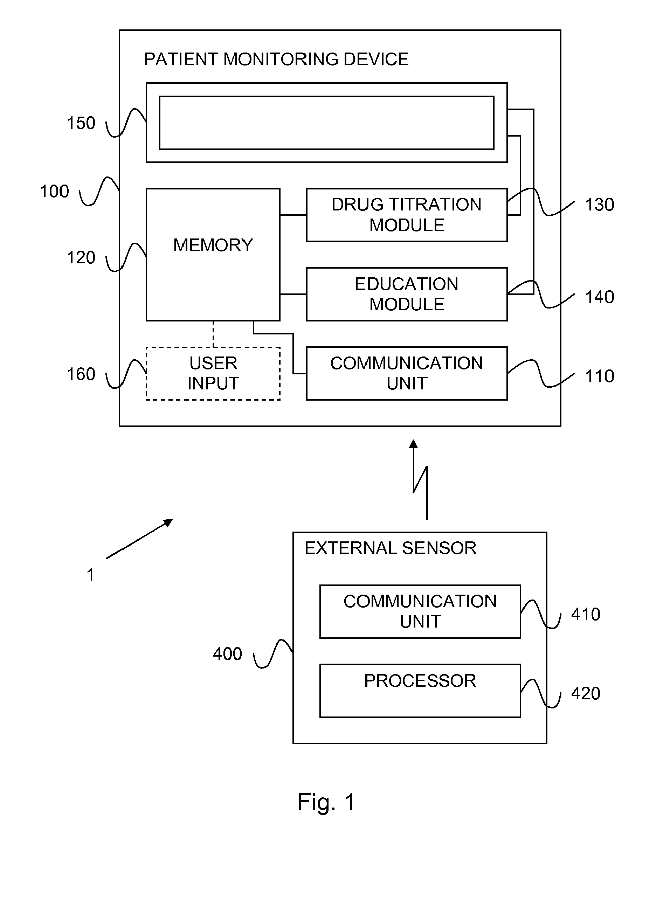

Fig. 1 is a schematic overview of a patient monitoring system 1 incorporating a patient monitoring device 100 according to an embodiment. The patient monitoring system 1 comprises at least one external sensor 400 configured to measure a medical or physiological characteristic or parameter of the patient. This medical characteristic can generally be any parameter that is to be used by the patient monitoring device 100 for, among others, determining a suitable dose of a prescribed drug. Hence, the particular external sensor 400 of the patient monitoring system 1 depends on various factors, such as the disease or medical condition of the patient, the particular drug or drugs prescribed to the patient and the drug titration algorithm currently used by the patient monitoring device 100. Non-limiting examples of external sensor embodiments that could be used in the patient monitoring system 1 include a weight scale, a blood pressure sensor, such as in the form of a blood pressure cuff, and a heart activity measuring sensor, such as in the form of a portable electrocardiogram (ECG) module. The particular medical characteristic of a patient that is determined by the external sensor 400 could, in these examples, be a current weight of the patient, a current blood pressure of the patient, a current heart rate or a current heart rate morphology of the patient.

The external sensor 400, irrespective of the particular sensory type and what medical characteristic that the external sensor 400 monitors, preferably comprises a processor 420 configured to generate a signal representative of a current value of the medical characteristic of the patient. The external sensor 400 also comprises a communication unit 410 configured to transmit this signal generated by the processor 420 to a corresponding communication unit 110 present in the patient monitoring device 100.

The communication between the external sensor 400 and the patient monitoring device 100 could be wireless, such as based on electromagnetic signals, such as radio frequency (RF), e.g. via Bluetooth® or WiFi, or infrared (IR) signals, inductive signals, etc., or wired communication. In the former case, the communication unit 410 of the external sensor 400 could be in the form of a transmitter or a transceiver, if bidirectional communication is desired. The corresponding communication unit 110 of the patient monitoring device 100 is then preferably implemented as a receiver or a transceiver. In the case of wired communication the communication unit 410 of the external sensor 400 could be a general output unit or a combined input and output (I/O) unit with the communication unit 110 of the patient monitoring device 100 as a matching input or I/O unit.

The patient monitoring device 100 of the embodiment illustrated in Fig. 1, thus, comprises a communication unit 110 configured to receive, from the external sensor 400, a signal representative of a current value of a medical characteristic of the patient. This current value is stored in a memory 120 connected to the communication unit 110.

The patient monitoring device 100 comprises at least two modules, units or devices that through their integration or interconnection provide a patient monitoring device 100 that can be used as a treatment adhering tool for the patient.

Thus, a drug titration module 130 is implemented in the patient monitoring device 100 connected to the memory 120. This drug titration module 130 is configured to process the current value stored in the memory 120 according to a drug titration algorithm available to the drug titration module 130 and preferably also stored in the memory 120. The processing of the current value using the drug titration algorithm generates information of a current dose of a prescribed drug. The drug titration module 130, hence, outputs this dose information. In a particular embodiment, the drug titration module 130 could generate the information of the current dose not only by processing the current value but also previous values from the external sensor 400, which is further disclosed herein.

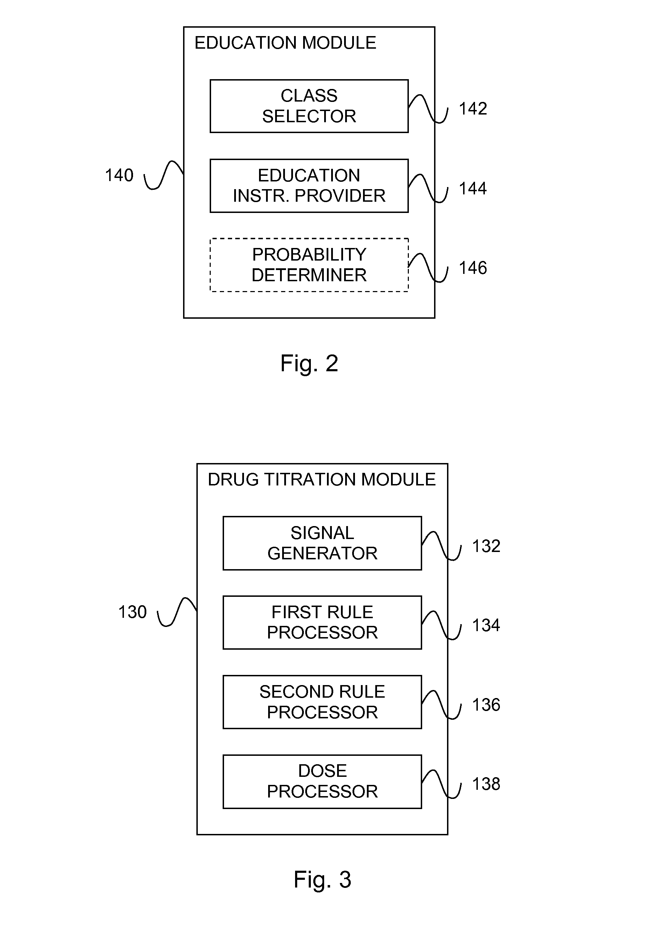

The patient monitoring device 100 also comprises an education module 140 connected to the memory 120. This education module 140 also processes and uses the current value stored in the memory 120. The education module 140 preferably comprises, which is schematically shown in Fig. 2, a class selector 142 and an education instruction provider 144. The class selector 142 is configured to select an education class among multiple education classes. Each such education class then comprises at least one, typically multiple different, education instruction(s). The class selector 142 performs this education class selection at least partly based on the current value of the medical characteristic of the patient. The education instruction provider 144 is then configured to provide, typically from the memory 120, an education instruction belonging to the education class selected by the class selector 142.

In a particular embodiment, the class selector 142 selects the education class not only based on the current value of the medical characteristic but also based on information of an education class previously selected by the patient, which is further described herein.

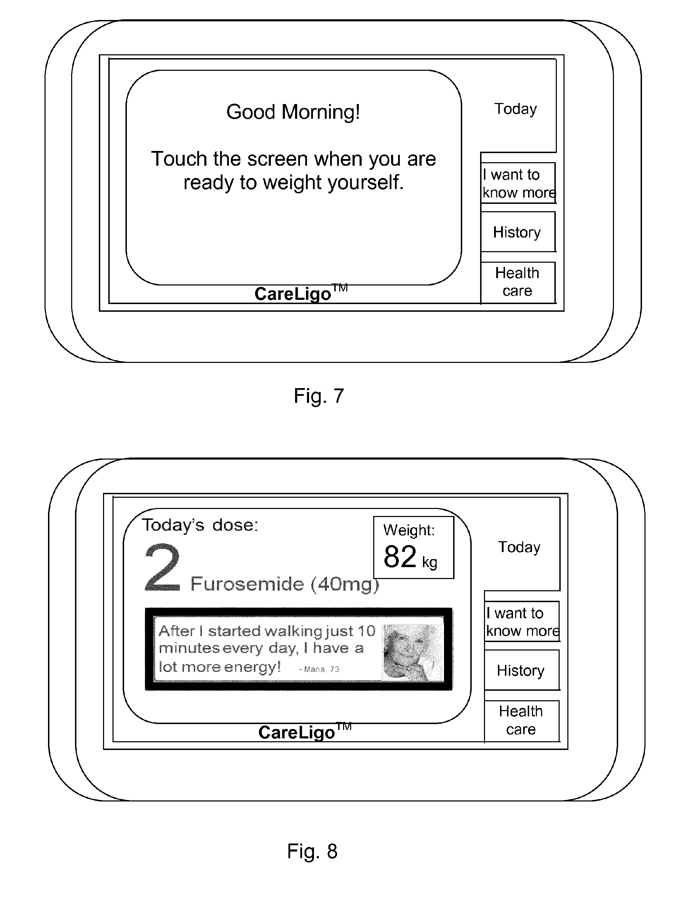

The patient monitoring device 100 also comprises a screen or display 150 configured to present, to the patient, the information of the current dose output by the drug titration module 130 and the education instruction provided by the education instruction provider 144 of the education module 140. Fig. 8 is an example of such a display of information of current dose and an education instruction.

The patient monitoring device 100 therefore determines and presents, on the screen 150, information of a current dose of a prescribed drug and an education instruction, both of which have been determined by the respective modules 130, 140 of the patient monitoring device 100 at least partly based on the current value of the medical characteristic of the patient as received from the external sensor 400.

Generally, lack of knowledge or understanding of the disease or medical condition of the patient correlates very well with low compliance and adherence to a prescribed treatment. This together with lack of correct management of drug administration by the patient is the main consequence of patient hospitalization and disease deterioration. The patient monitoring device 100 solves these problems by determining suitable drug doses and education instructions or "tip of the day" that are based on the current patient situation as represented by the sensor output from the external sensor 400. This means that the patient monitoring device 100 is capable of providing the most appropriate information, i.e. drug dose and education instruction, for the current condition of the patient. The information is preferably provided to the patient in a simple and intuitive way as shown in the example of Fig. 8. This means that the information is easily accessible and understandable for the patient, and preferably conveyed by another patient with similar diagnosis and demography.

A module 130, 140 connected to the memory 120 could be implemented by a direct or indirect electrical connection between the memory and the module 130, 140. Alternatively, the module 130, 140 is implemented in software stored in the memory 120 and run by a processing unit of the patient monitoring device 100.

Fig. 3 illustrates various units of the drug titration module 130. In an embodiment, the drug titration module 130 first verifies whether the current value retrieved from the memory is reasonable and not a measurement error. This verification reduces the risk of determining inappropriate drug doses by the drug titration algorithm based on incorrect input values. In an embodiment the drug titration module 130 therefore comprises a signal generator 132 configured to verify whether the current value is within a target value range. If the current value is within the target value range it is likely that the current value correctly represents the current medical characteristic of the patient as determined by the external sensor and a correct drug dose can therefore be determined. However, if the current value is outside of the target value range it is likely that the current value is due to some measurement error and this current value should not be used to determine a drug dose.

In an embodiment the signal generator 132 is configured to generate a blocking signal if the current value is outside of the target value range. The drug titration module 130 is then responsive to the blocking signal and is configured to block processing of the current value according to the drug titration algorithm based on the blocking signal. Hence, no drug dose is determined based on this incorrect current value.

In an optional embodiment, the incorrect current value is removed from the memory so that it no longer will be used by the drug titration module 130 if the signal generator 132 generates a blocking signal.

In an alternative approach the signal generator 132 is configured to generate a proceed signal if the current value is within the target value range. In such a case the drug titration module 130 only processes the current value according to the drug titration algorithm if the signal generator 132 generates this proceed signal.

These two alternative embodiments can be combined so that the signal generator 132 generates, each time a new current value is received from the external sensor, either a proceed signal or a blocking signal depending on the current value. The drug titration module 132 thereby either blocks processing of the current value or proceeds with the processing of the current value depending on whether the signal generator 132 outputs the blocking signal or the proceed signal.

The comparison of the current value and the target value range could be a simple comparison of the current value with a single threshold value. Thus, if the current value exceeds (or is below) the threshold value it is regarded as being within the target value range. In an alternative approach the target value range is defined by a minimum threshold value and a maximum threshold value so that the signal generator 132 then compares the current value with these multiple threshold values. In the latter case this range could be defined as ±Y % of an initial value of the medical characteristic of the patient. The value Y could be preconfigured or set by the physician.

The drug titration module 130 could have access to multiple drug titration algorithms, such as stored in the memory 120. In this approach the patient monitoring device 100 is configured to be used in connection with different patient groups, each having at least one suitable drug titration algorithm implemented in the memory 120. The patient's physician can then use the patient monitoring device 100 to select which particular drug titration algorithm to use for the present patient. In such a case, the patient monitoring device 100 comprises a user input 160 configured to receive an algorithm selection signal. This user input 160 could be in the form of a key that is pressed or activated by the physician. Alternatively, the screen 150 could be in the form of a touch-sensitive screen 150. In such a case, the physician can activate the user input 160 by pressing on a selected portion of the touch-sensitive screen 150. The user input 160 could also be in the form a receiver configured to receive an algorithm selection signal from a (wirelessly or wiredly) connected keyboard, mouse or other device.

The drug titration module 130 is then configured to select one of the multiple drug titration algorithms stored in the memory 120 based on the algorithm selection signal.

The user input 160 mentioned in the foregoing can also or alternatively be used by the physician to input information to calibrate the (selected) drug titration algorithm for the particular patient. For instance, the physician can use the user input 160 to provide information of a starting dose of the prescribed drug, information of an incremental dose increase and/or information of a day number. The drug titration module 130 is then configured to define the drug titration algorithm based on the received information. In this particular example, the starting dose represents the initial dose of the prescribed drug. Incremental dose increase represents the increase in the dose of the prescribed drug that is desired in response to a defined change in the current value received from the external sensor 400 as compared to a default value or a previously received value of the medical characteristic of the patient. The optional day number could be set to define which previous value to compare with the current value from the current day, such as the previous value from yesterday, the previous value from the day before yesterday, and so on, or an output value from a mathematical operation performed on the previously stored values.

The drug titration algorithm is preferably run by the drug titration module 130 once the patient monitoring device 100 receives the signal representative of the current value of the medical characteristic of the patient. Hence, the patient monitoring device 100 preferably automatically determines the information of the current dose of the prescribed drug once it receives the signal from the external sensor 400.

In addition, if the physician sets the control parameters of the drug titration algorithm as disclosed above using the user input 160 the patient's drug regimen will be tailor-made for that specific patient and will be the physician's recommended dose every day, given the current status of the patient.

A particular example will now be presented of how a drug titration algorithm can be defined based on received information input by the physician using a user input 160 of the patient monitoring device 100. In this particular example, the patient is a heart failure patient and the prescribed drug is a diuretic. The external sensor 400 is exemplified by an external weight scale with a sensor configured to generate a signal representative of a current value of a weight of a patient standing on the weight scale. The drug titration module 130 then processes the current value and information of a starting dose, stored in the memory 120, according to the drug titration algorithm to output the information of the current dose of the diuretic.

In an embodiment of this example the drug titration module 130 comprises a first rule processor 134 configured to output a first rule parameter having a first value if Wₙ-Wₙ₋₁≥X₁ and having a second value if Wₙ-Wₙ₋₁<X₁. In this example Wₙ represents the current value of the weight at day n, Wₙ₋₁ represents a previous value of the weight at day n-1 stored in the memory 120 and X₁ is a first threshold value stored in the memory 120. The drug titration module 130 also comprises a second rule processor 136 configured to output a second rule parameter having the first value if Wₙ-Wₙ₋₃≥X₂ and having the second value if Wₙ-Wₙ₋₃<X₂. In this example Wₙ₋₃ represents a previous value of the weight at day n-3 stored in the memory 120 and X₂ is a second threshold value stored in the memory 120.

A dose processor 138 of the drug titration module 130 is configured to determine the current dose, Dₙ, of the prescribed drug, preferably the diuretic, to be equal to one of i) to v) below:
i) the starting dose if the memory 120 does not store the previous value of the weight at day n-1;
ii) the starting dose plus an incremental dose if the memory 120 does not store the previous value of the weight at day n-3 but stores the previous value of the weight at day n-1 and the first rule parameter has the first value;
iii) the starting dose if the memory 120 does not store the previous value of the weight at day n-3 but stores the previous value of the weight at day n-1 and the first rule parameter has the second value;
iv) the starting dose plus an incremental dose if the memory 120 stores the previous values of the weight at day n-1 and day n-3 and the first rule parameter has the first value and/or the second rule parameter has the first value;
v) the starting dose if the memory 120 stores the previous values of the weight at day n-1 and day n-3 and the first rule parameter has the second value and the second rule parameter has the second value.

The memory 120 stores the starting dose and the incremental dose, both of which have preferably been set by the physician using the user input 160 as previously discussed.

The above present example is based on the European Society of Cardiology (ESC) guidelines that propose that a 2 kg increase in the weight of the patient over 3 days should trigger a dose increase and the American College of Cardiology/American Heart Association (ACC/AHA) guidelines suggesting that a 2 Ibs increase in weight over one day should trigger a dose increase.

The drug titration algorithm mentioned above can then be defined as:

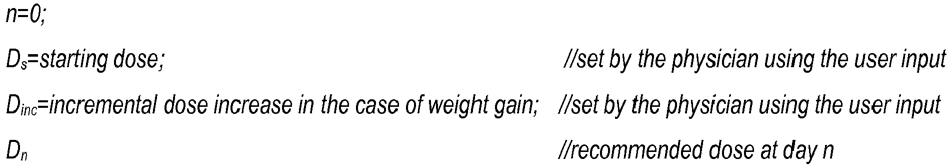

The first rule processor 134 could then implement the ACC/AHA requirements with the threshold X₁=1 (2 Ibs rounded to 1 kg):

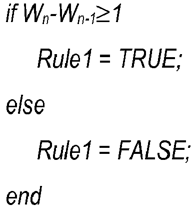

The second rule processor 136 could then implement the ESC requirements with the threshold X₂=2:

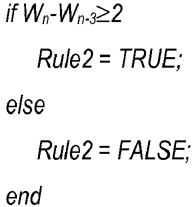

The dose processor 138 will then use these two Boolean variables (TRUE and FALSE) to decide if the dose should be up-titrated or not based on a logical OR function between these two rules. In other words, as long as one of the rules is TRUE the diuretics should be up-titrated. In an optional case, the drug titration module 130 outputs the increased dose for e.g. two days regardless of what happens with the weight of the patient the day after the increase. This is of course optional and could be selected by the physician using the user input 160.

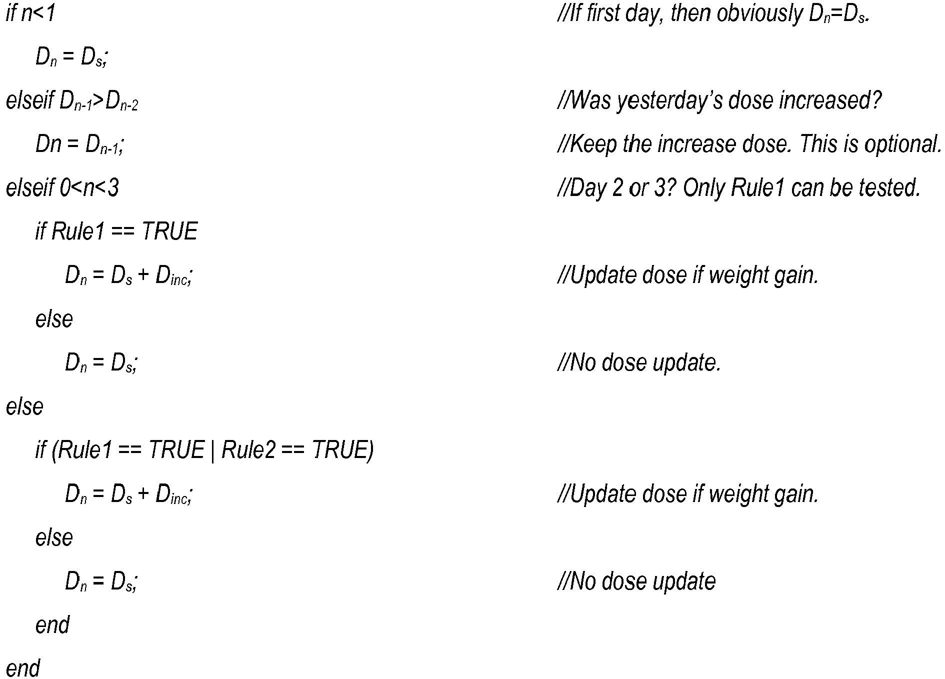

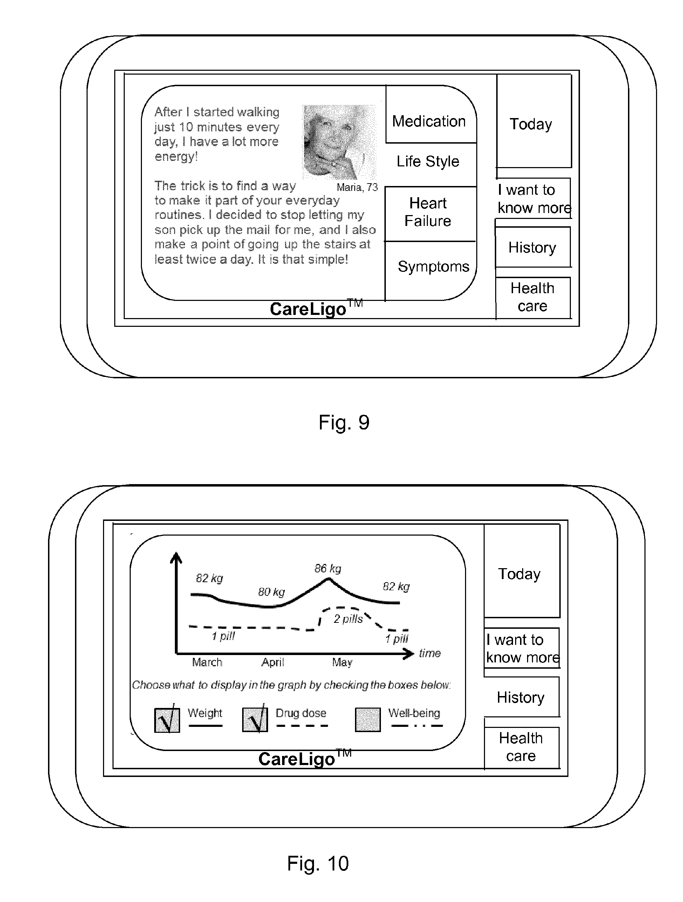

The education module 140 of the patient monitoring device 100 preferably selects the education class not only based on the current value of the medical characteristic of the patient. In preferred embodiments also other parameters could be used in the class selection. Fig. 9 illustrates the screen 150 of the patient monitoring device 100 when the education instruction or tip tab has been selected by the patient. The figure illustrates four examples of different education classes: medication education class, lifestyle education class, disease, here represented by heart failure, education class and symptoms education class. The disease/heart failure education class then comprises at least one education instruction with information relating to the (heart failure) disease the patient is suffering from. The medication education class comprises at least one education instruction with information relating to any drugs prescribed to the patient and the lifestyle education class comprises at least one education instruction with information relating to a lifestyle changing tip to improve the disease status of the patient. The symptoms education class comprises education instructions relating to symptoms of the particular disease.

The patient can then, using the user input 160, select one of these education classes. The user input 160 generates a class selection signal representative of the education class selected by the patient by activating the user input 160. The education instruction provider 144 of the education module preferably provides one of the education instructions belonging to the selected education class and presents this education instruction on the screen 150 of the patient monitoring device 100. In an embodiment, the education instruction provider 144 could randomly select one of the education instructions belonging to the selected education class. In another embodiment, the education instruction provider 144 step-by-step goes through the education instructions in each education class. This means that the first time the patient selects a particular education class the education instruction provider 144 preferably provides and presents the first education instruction of that education class, the second time this education class is selected the second education instruction is provided and so on. Once all education instructions of an education class have been provided, the education instruction provider 144 could return to once more presenting the first education instruction to start a new cycle.

In a particular embodiment, each education class comprises multiple, i.e. at least two, different education instructions.

The education instruction provider 144 could be configured to present the education instruction in another way than what is shown in Fig. 9. For instance, words could be highlighted in a short text and activating such a highlighted word using the user input 160 causes the education instruction provider 144 to provide an education instruction similar to hyper links.

The above disclosed function of the education module 140 in providing and presenting education instructions is activated once the patient itself activates the education tab. However, the education module 140 can in an embodiment also use this information of previously selected education classes when automatically selecting an education class and providing an education instruction in connection with presenting the information of the drug dose, see Fig. 2.

In this embodiment the memory 120 therefore stores information of education classes previously selected by the patient, i.e. stores information based on the selection signal generated when the user activates the user input 160 to select one of the education classes. The class selector 142 is then configured to select the education class at least partly based on the current value of the medical characteristic of the patient and the information of the previously selected education classes.

This means that in this approach the education class is selected not only based on the current medical characteristic and condition of the patient, as represented by the current value from the external sensor 400, but also based on the preferences of the patient. Thus, if the patient is more interested in one of the education classes and therefore previously has selected this education class more often than other education classes, the class selector 142 preferably weights this education class heavier than other education classes during the selection. The reason for this is that it is more beneficial to present information to the patient that he/she is interested in to encourage him/she to adhere to the prescribed treatment than to present information that the patient is more likely to ignore.

In an embodiment the education module 140 therefore comprises a probability determiner 146 configured to determine a respective selection probability for each education class based on the current value and based on the information of previously selected education classes. For instance, depending on the current value it might be more beneficial to select one of the education classes over the other ones. In such a case, this education class preferably gets a higher selection probability. Correspondingly, if the patient is particularly interested in one of the education classes this one preferably gets a higher selection probability than the other education classes. The selection probability could, in an example, be represented by weights, such as one weight per education class. Each such weight could then be a product of two terms, one of which is determined based on the current value and the other based on the patient's preferences of education classes. The higher weight an education class has the more likely this education class is selected by the class selector 142.

Another implementation of this pseudo-random selection of education classes could be that out of *N* consecutive education instructions that are presented along with the determined drug dose *M* education instructions are picked from the education class which the patient has studied the most times, where *M*<*N*. The particular values of *M*, *N* can be preprogrammed in the patient monitoring device 100.

The class selector 142 is in this embodiment configured to pseudo-randomly select the education class among the multiple predefined education classes based on the selection probabilities determined by the probability determiner 146. The education instruction provider 144 then provides one of the education instructions belonging to this education class as previously determined herein, such as randomly selecting one of the education instructions or providing the next education instruction according to a defined order.

Also other parameters besides the current value and user preferences with regard to education classes could be used by the class selector 142 when selecting an education class. For instance, information of the particular time during the day at which the patient performs the sensor readings to get the current value of the medical characteristic and/or the frequency at which the patient performs the sensor readings could be used in the selection of education classes.

The education module 140 of the patient monitoring device 100 provides education to the patient in a specific manner anchored in behavioral science and tailored for the specific patient. The education module 140 is, as mentioned above, preferably intelligent and learns what the patient is interested in and focuses on conveying information in a way that is perceived as positive and enabling for the patient. The education module 140 therefore provides this information in a simple tip of the day format, in connection with presenting the determined drug dose. This optimizes understanding of the education instructions. In addition, the education module 140 preferably enables the patient to manually select and present additional education instructions if desired.

Lack of knowledge or understanding of the patient's medical condition correlates very well with low compliance to a prescribed treatment. Correspondingly, high levels of understanding and knowledge predict better response to the treatment. In fact, not only compliance with respect to adherence to drug treatment but also outcomes are positively influenced by training and education.

The education module 140 of the patient monitoring device 100 provides this information in a manner and format that is easily perceived and user-friendly.

The education module 140 preferably automatically presents the education instruction in connection with presenting the determined drug dose. This education instruction is advantageously in a format that tells the patient what it can do instead of what it cannot do, i.e. in a positive manner. The education instruction is preferably presented by another patient suffering from the same disease as the current patient as indicated in Figs. 8 and 9. This approach has been shown to be a powerful means to increase education uptake. Thus, the education instructions are preferably given in a positive manner emphasizing possibilities rather than focusing on what the patient should not do. The education instructions are preferably given as comments or suggestions rather than orders or directives and/or are preferably given by a fellow patient in the same or similar situation.

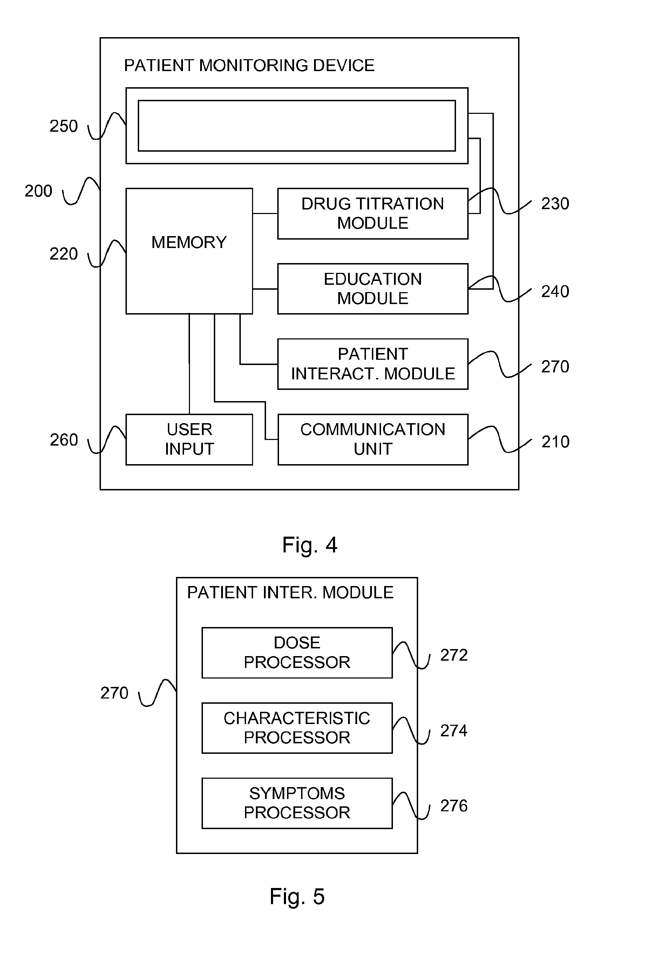

The patient monitoring device 200 may, as shown in Fig. 4, also have a patient interaction module 270 in addition to the modules or units presented in Fig. 1. This patient interaction module 270 enables the patient to interact with the patient monitoring device 200 and obtain information of his/her medical history. In such a case, the memory 220 of the patient monitoring device 200 preferably stores the information of the current dose as determined by the drug titration module 230 based on the current value of the medical characteristic obtained from the external sensor 400. The patient interaction module 270 comprises, as shown in Fig. 5, a dose processor 270 configured to process the information of the current dose to generate information representing current dose over time for the patient. Fig. 10 illustrates such information graphically presented on the screen 250 of the patient monitoring device 200. The patient interaction module 270 also comprises a characteristic processor 274 configured to process the current value of the medical characteristic to generate information representing current value of the medical characteristic over time for the patient.

In such a case, the patient can use the user input 260 of the patient monitoring device 200 to select one of multiple interaction types. Fig. 10 illustrates this concept by showing three alternative choices: "weight", "drug dose", and "well-being". The user input 260 then generates an interaction signal representative of the selected interaction type(s). The patient interaction module 270 is responsive to this interaction signal and is configured to present, based on the interaction signal, one of the information generated by the dose processor 272 and the information generated by the characteristic processor 274 on the screen 250.

Hence, the patient interaction module 270 enables the patient to follow the determined drug dose over time and/or his/her medical characteristic over time. The patient interaction module 270 is therefore capable of feeding information relating to the health status and dose prescription right back to the patient to enable self-assessed symptoms tracking and health status and medication trends.

The patient interaction module 270 could also include additional processors as illustrated by the symptoms processor 276 in Fig. 5. This symptoms processor 276 is configured to present, on the screen 250, information urging the patient to enter a current symptom of the patient. This could be in a simple format such as selecting between feeling fine, ok versus bad to more detailed symptom information. The patient then uses the user input 260 to generate a symptoms signal representative of the current symptom entered by the patient. The symptoms processor 276 stores this symptoms signal in the memory 220.

In a particular embodiment the symptoms processor 276 generates a hospitalization signal based on the symptoms signal, the current value and information of a previous hospitalization period of the patient stored in the memory 220. Thus, the patient monitoring device 200 then stores information of a previous period when the patient was hospitalized due to deterioration of his/her health status. This information is stored together with the symptoms signal and the current value(s) stored in the memory 220 during and/or immediately prior to the hospitalization period. The symptoms processor 276 could then compare the current symptoms signal and current value with the corresponding symptoms signal and value of the medical characteristic that preceded the previous hospitalization occasion. If the current signal and values are very similar to the previous signal and values there is a risk for a forthcoming new hospitalization period for the patient. The patient monitoring device 200 could then present to the patient and based on the hospitalization signal generated by the symptoms processor 276 a warning of a risk for hospitalization.

Herebelow a typical use of the patient monitoring device and system of the embodiments will be further discussed in connection with such a device and system adapted for a heart failure (HF) patient.

The HF patient is prescribed the patient monitoring system of the embodiments by a managing physician, which is installed directly in the patient's home. In addition, the HF patient preferably brings the patient monitoring device of the system to the physician at follow-up or visits at the healthcare facility of the physician. In such a case, the physician can, at the first such visit, input any required information, such as selection of drug titration algorithm and inputting starting parameters of the drug titration algorithm. In another embodiment the patient monitoring device can be accessed via mobile phone lines, the internet, or by other means, by the physician so that the patient does not have to bring his/her patient monitoring device to the physician.

Below a typical or normal every-day flow of the patient monitoring system in use at the patient's home is described.

The patient wakes up and steps onto the weight scale, which constitutes the external sensor of the patient monitoring system in this particular example. The patient monitoring device may optionally present a welcome message on the screen as indicated in Fig. 7. The patient's weight is automatically transmitted wirelessly to the patient monitoring device, where the drug titration algorithm will decide, based upon the conditions preferably set by the physician, what today's diuretics dosage should be. In connection with presenting the determined dosage, such as immediately prior to, together with or immediately following presentation of the dosage, the patient monitoring device also presents a "tip of the day", i.e. an education instruction, which appears in a personal manner to educate the patient. The determined dosage of diuretics, given the patient's unique weight trend and doctor recommendation, is also given on screen, see Fig. 8.

This concludes the daily procedure and will take approximately 1 minute of the patient's time. This is very simple for the patient, since nothing is to be found after it was misplaced, nothing is to be noted anywhere, nothing is to be compared with anything that they were supposed to remember - everything is fully automated and neatly served to them in an efficient manner.

However, if the patient so desires, he or she can with the flick of a finger move on to learn more about whatever this particular patient is most interested in through the incorporation of the education module and preferably the patient interaction module of the patient monitoring device, see Figs. 9 and 10.

If the managing physician has chosen to activate this feature, the patient will also be asked to assess his/her symptoms today, or rather describe how he/she is feeling by choosing the appropriate symbol, presented on the screen.

If the patient is interested in seeing how he or she is doing, with the simple touch on the screen the patient will be presented with charts displaying the long-term and short-term weight variations through the action of the patient interaction module. How the dosage of diuretics has varied over time is also presented. If activated, the patient may also see how his/her well-being has changed over time and tie that to the other factors presented to the patient, thereby further enhancing feedback to the patient and empowering the patient, see Fig. 10.

Herebelow follows different examples of how the modules of the patient monitoring device will co-interact and perform their intended functions based on output signal from the external sensor. In these example, the patient is assumed to be a HF patient and the drug titration module is configured to output information of a current dose of a prescribed diuretic.

If a patient's nominal diuretics dose is zero, and the diuretics will only be prescribed following a weight-increase, then it may be important to make sure that the "tip of the day" of that day is a description of what diuretics are, i.e. a tip from the medication-tag set. Thus, in this case the drug titration module uses a drug titration algorithm set, preferably by the physician, to have a starting dose equal to zero. However, if the signal received from the weight scale indicates a weight increase above a set, preferably by the physician, a threshold value, the drug titration module outputs information that a non-zero dose of the diuretics should be taken by the patient that day. In addition, the class selector of the education module is configured to select, based on the increase weight, the medication education class to thereby enable the education instruction provider to provide and present an education instruction giving valuable information to the patient about diuretics.

In an embodiment the class selector could select the particular education class to be an education class comprising the most vital information and instructions if the patient does not regularly weighs himself/herself. In such a case, the education module guarantees that at least the most important education instructions are presented to the patient when he/she eventually uses the patient monitoring system. In a particular embodiment, the physician could use the patient monitoring device to define or select which education class/classes that is/are regarded as being the most important.

In an embodiment, if the patient performs the weighing procedure at very varying times in the morning, e.g. spread out from between 4 a.m. and 10 a.m. it is likely that the patient is suffering from sleep disorder. Sleeping tips tailored for heart failure patients could then be included in an education class. The class selector could then use this information of the particular time at which the patient monitoring device receives the sensor signal to determine whether education instructions taken from the sleeping tips education class would be beneficial to the patient or not.

In an embodiment, if a slowly increasing weight is detected, i.e. over weeks/months rather than days, then it is likely the patient is gaining fatty tissue. The class selector is then preferably configured to select education classes containing education instructions relating to diet and exercise, e.g. from a lifestyle education class.

In an embodiment, if the patient starts to weigh himself/herself more often and at the same time a general deterioration in self-reported symptoms is detected, then the patient monitoring device could present a warning on the screen encouraging the patient to contact his/her physician.

In an embodiment, if the frequency with which the patient uses the patient monitoring system goes down, and symptoms deteriorate as well as other types of interactions, the class selector could select an education class with education instructions informing the patient about what generally occurs prior to a hospitalization and thereby encourage physician interaction.

In an embodiment, if the "symptoms" feature is activated by the physician, but the patient is not using it to input his/her symptoms, the class selector could select an education class with education instructions educating the patient on how important it is, using the tip of the day function.

In an embodiment, if the patient utilizes the option to input the date of any potential hospitalizations that occur, then a compound image of what led up to the hospitalization in terms of symptoms, dose, weight etc. can be displayed to the patient so as to teach the patient what the warning signs are for imminent deterioration or hospitalization.

Fig. 10 illustrates an example of the display screen when the patient has activated the patient interaction module in order to present information of the patient's medical history as recorded by the patient monitoring device. The so-called "history-tab" of the screen enables the patient to select, in this example among three options, which are "drug dose", "weight" and "well-being". There is also the possibility to overlay all three parameters to make the connections between the trends, e.g. "here I can see that my weight increased, I started to feel ill, then the dose was increased, the weight dropped and I started feeling better again" or "I can see my weight has dropped and I am feeling better since I started walking like I read about in that tip! I wonder what else I can do to improve my well-being - let's go to the tips-tab!".

The patient monitoring device preferably stores every weight and every dose as well as every input symptom characterization (if that option is used) and they can be viewed by the patient at any time. This is a powerful way to get the patient to take control over his or her health care.

Several other options are plausible here and the embodiments are not limited by what was just described. For instance it could rather easily be implemented to give the patient the possibility to make plans for diets and losing weight, for instance, and the system could help the patient follow up on that. Similarly an exercise module could be added to either have the patient input heart rates (or read them from a pulse watch automatically) or simply the dates of exercise which can then later be correlated to weight loss and/or change of symptoms.

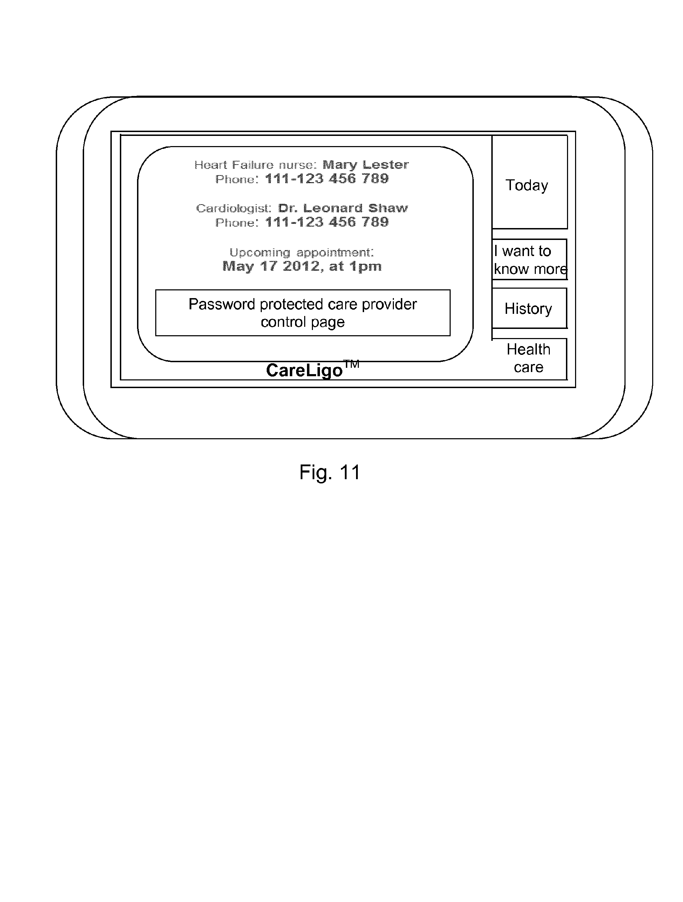

The patient monitoring device of the embodiments is very flexible allowing the patient's physician to take an active part in defining the operation of the modules. For instance, the physician can use the patient monitoring device to set up diuretics parameters for the patient in question by selecting and/or defining the parameters of the drug titration algorithm. This means that the physician can, among others, input the starting dose for the patient and select which drug titration algorithm or rule to use. In an example, a default setting could be the logical OR combination of both the American and European guidelines disclosed above. The physician can however, chose to use anywhere between one or several, and for each of these rules (unless the default are selected) the physician will specify what weight change, over what time period, that will result in what dose change (+ or - x pills). This means that the physician can use the patient monitoring device to create a patient-specific drug titration algorithm. In such a case, the physician preferably sets, in addition to the above mentioned parameters, maximum dose for the patient (as a safety measure used by the patient monitoring device to avoid over-prescribing of the patient). In addition, the physician can elect to input contact information to the caregiver (and information about next follow-up). There may also be a free text entry, where the physician may enter information about how the patient should contact his/her caregiver, the date of the next follow-up which in turn can be connected to an alarm-function to further assist the patient, or anything else that could be useful for the patient to always have available, see Fig. 11

The physician can also use the patient monitoring device to define which features of the patient monitoring system that are mandatory or not for the particular patient. This can be achieved by having various check-boxes that are selected by the physician. For instance:
- Check-box 1: Should the patient be told to contact the caregiver as soon as the patient monitoring system has increased the dosage? "Yes"/"No"
- Check-box 2: Should a patient after a dose increase remain on that new dose for an additional time, regardless of what weight the patient displays those days? "1 day"/"2 days"/"No"
- Check-box 3: Should the symptom-monitoring feature be turned on? "Yes"/"No"
- Check-box 4: Should an alarm be set to alert the patient of an imminent follow-up? "Yes + date"/"No"

All of these data could be input in table form for ease of use.

In particular embodiments, the physician may also be able to identify the patient with a few characteristics such as age and gender etc. This information can be used by the patient monitoring system to access the right patients to be the ones that appear giving the advice, tips and stories in the "tip of the day" section of the patient monitoring device.

The graphical user interface (GUI) of the patient monitoring device is preferably designed adapted to the particular patient group that will use the patient monitoring device. For instance, the average person who is diagnosed with heart failure is over 75 years old and usually has little or no experience of computers. The GUI is therefore preferably designed so to resemble the classic catalogues that people have been using for a century and it is a very intuitive and unique design that makes the adoption of this technology simpler. A rough sketch of the GUI can be seen in any of the pictures in Figs. 7-11. The principle is to use tabs, such as colored tabs, in the margin so to remind the user of going through for instance a phone book or any indexed binder when looking for something and the color coding makes it very easy to understand where you are.

Also other solutions for the GUI besides using tabs are possible and within the scope of the embodiments.

In a particular embodiment as little information as possible is preferably displayed during the everyday use of the patient monitoring device, to not discourage non-computer experienced users. For instance, Fig. 8 illustrates that the information of the current dose is simply displayed as the number of pills to be taken of the prescribed drug.

The patient monitoring system is intentionally designed to be modular in its construction. This implies that virtually any kind of sensor can be connected to the patient monitoring device, to either improve the care for the (heart failure) patients or broaden the patient population that may benefit from the patient monitoring system and expand it beyond heart failure. For instance, a blood pressure cuff that could wirelessly communicate with the patient monitoring device could help the analysis of the status of the heart failure patient but also be valuable for any patient with high blood pressure. The blood pressure readings could be used to titrate blood pressure medicines using the same principle as described herein for diuretics. The values could be trended and displayed for the patient and obviously the education module could be updated to include hypertension-related information as well. Yet another example would be to use some kind of ECG module to look for atrial fibrillation (AF) or other rhythm disorders and the same principles apply there. It could titrate warfarine, show trends in arrhythmia behavior and educate the patient. These are only a few examples of how this patient monitoring system could be expanded and should not be viewed as an exhaustive list, but merely an illustration of how the patient monitoring system is intended to be used to aid as many patients as possible.

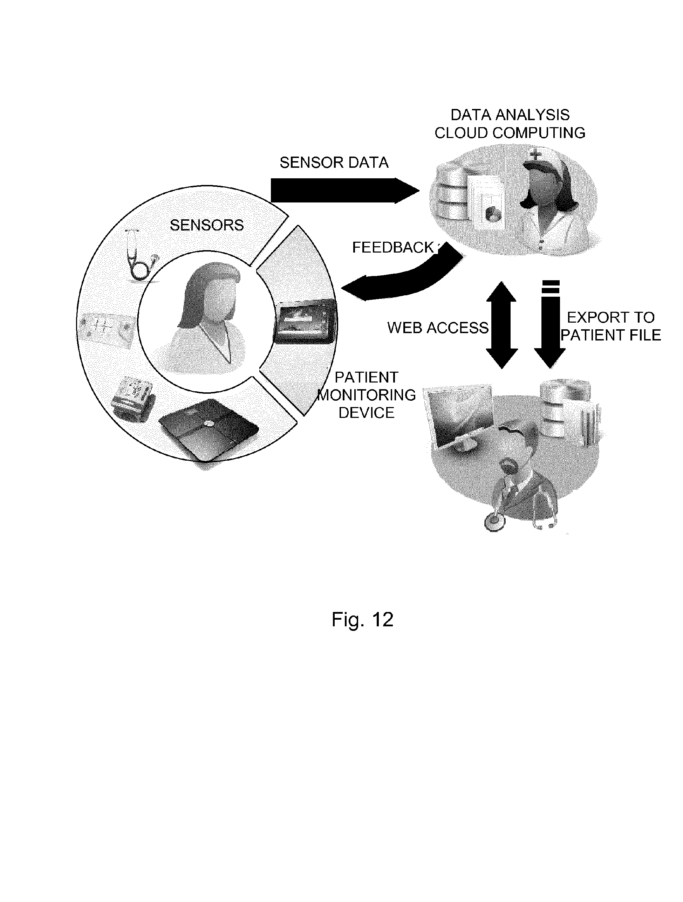

Fig. 12 schematically illustrates this concept by showing several sensors that can interact with the patient monitoring device. The patient monitoring device could also be connected to a healthcare facility as shown in the figure to forward the collected sensor data to the healthcare facility. This enables the physician to get up-to-date data of the patient status and may also enable detection of radical deteriorations in health. All the data from the patient monitoring device may also be stored centrally. In such a case, external expert systems using more advanced algorithms than what are implemented by the modules in the patient monitoring device could be used to process the patient data as indicated in the figure.

In the foregoing, the patient monitoring device has mainly been described in connection with automatically determining a current dose of a prescribed drug based on the output signal from the external sensor. The patient monitoring device can, however, also be used in cases where the patient is prescribed multiple different drugs or medicaments. In such a case, the optimal dose of all or at least one of these could be determined by the drug titration module, possibly using a respective drug titration algorithm for each drug, based on the current value of the medical characteristic of the patient. It could also be possible that some of the drugs should be taken at a dose that is not dependant on the current value. In such a case, the memory of the patient monitoring device could store this fixed or semi-static (if the physician is able to program a new dose value in the memory) dose. A further variant is to have a patient monitoring system comprising multiple external sensors measuring different medical characteristics of the patient. In such a case, the dose of at least a first drug could be dependent on the current value from a first external sensor, whereas the dose of at least a second drug is dependent on the current value from a second external sensor or indeed dependent on the current values from both external sensors. The drug titration module then has access to different drug titration algorithms that operate on the respective current values from the external sensors to output determined current doses of the drugs. In all these embodiments the screen preferably presents the respective dose, either calculated by the drug titration module or simply retrieved from the memory, to the patient. Hence, in a preferred embodiment the patient monitoring device provides the patient with all the drug dose information needed in order to comply with the treatment plan of the patient.

The patient monitoring device can be implemented in hardware, in software or a combination of hardware and software. The patient monitoring device can be implemented as a notebook, tablet or other form of, preferably portable, user equipment.

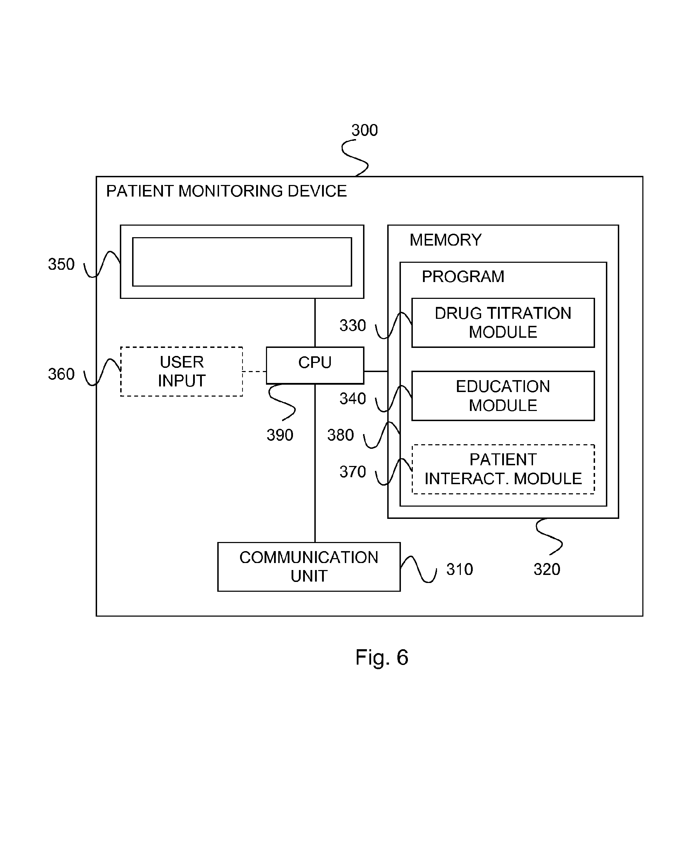

Although the respective unit disclosed in conjunction with Figs. 1-5 have been disclosed as physically separate units in the patient monitoring device, and all may be special purpose circuits, such as ASICs (Application Specific Integrated Circuits), alternative embodiments of the patient monitoring device are possible where some or all of the units are implemented as computer program modules running on a general purpose processor. Such an embodiment is disclosed in Fig. 6.

Fig. 6 schematically illustrates an embodiment of a patient monitoring device 300 comprising the previously mentioned screen 350, communication unit 310, optional user input 360 and memory 320. The patient monitoring device 300 also comprises a processing unit 390, such as a DSP (Digital Signal Processor) or CPU (Central Processing Unit). The processing unit 390 can be a single unit or a plurality of units for performing different functions described herein.

Furthermore, the patient monitoring device 300 comprises at least one computer program product in the form of a non-volatile memory 320, for instance an EEPROM (Electrically Erasable Programmable Read-Only Memory), a flash memory or a disk drive. The computer program product comprises a computer program 388, which comprises code means which when run on or executed by or on the patient monitoring device 300, such as by the processing unit 390, causes the patient monitoring device 300 to perform the functions described in the foregoing in connection with Figs. 1 and 4. Hence, in an embodiment the code means in the computer program 380 comprises a drug titration module 330, an education module 340 and optionally a patient interaction module 370. These modules 330, 340, 370 essentially perform the functions of corresponding modules in Figs. 1 and 5 when run on the processing unit 390.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

## Claims

1. A patient monitoring device (100, 200, 300) comprising:
a communication unit (110, 210, 310) configured to receive, from an external sensor (400), a signal representative of a current value of a medical characteristic of a patient;
a memory (120, 220, 320) connected to said communication unit (110, 210, 310) and configured to store said current value;
a drug titration module (130, 230, 330) connected to said memory (120, 220, 320) and configured to process said current value according to a drug titration algorithm, stored in said memory (120, 220, 320), to output information of a current dose of a prescribed drug;
a screen (150, 250, 350) configured to present, to said patient, information of multiple predefined education classes, wherein each education class of said multiple predefined education classes comprises at least one education instruction;
a user input (160, 260, 360) configured to generate a class selection signal representative of an education class selected by said patient by activating said user input (160, 260, 360), wherein said memory (120, 220, 320) is configured to store, based on said selection signal, information of said education class selected by said patient; and
an education module (140, 240, 340) connected to said memory (120, 220, 320) and comprising:
a class selector (142) configured to select, at least partly based on said current value and said information of said education class selected by said patient, an education class among said multiple predefined education classes; and
an education instruction provider (144) configured to provide, from said memory (120, 220, 320), an education instruction belonging to said education class selected by said class selector (142), wherein said screen (150, 250, 350) is configured to present, to said patient, said information of said current dose and said education instruction provided by said education instruction provider (144).

2. The patient monitoring device according to claim 1, wherein
said education module (140) comprises a probability determiner (146) configured to determine a respective selection probability for each education class of said multiple predefined education classes based on said current value and said information of said education class; and
said class selector (142) is configured to pseudo-randomly select said education class among said multiple predefined education classes based on said selection probabilities determined by said probability determiner (146).

3. The patient monitoring device according to claim 1 or 2, wherein
said drug titration module (130) comprises a signal generator (132) configured to generate a blocking signal if said current value is outside of a target value range and/or generate a proceed signal if said current value is within said target value range; and
said drug titration module (130) is responsive to said blocking signal and/or said proceed signal and is configured to block processing of said current value according to said drug titration algorithm based on said blocking signal and/or proceed to process said current value according to said drug titration algorithm based on said proceed signal.

4. The patient monitoring device according to any of the claims 1 to 3, further comprising a user input (160, 260, 360) configured to receive information of a starting dose, information of an incremental dose increase and information of a day number, wherein said drug titration module (130, 230, 330) is configured to define said drug titration algorithm based on said information of said starting dose, said information of said incremental dose increase and said information of said day number.

5. The patient monitoring device according to any of the claims 1 to 4, further comprising a user input (160, 260, 360) configured to receive an algorithm selection signal, wherein
said memory (120, 220, 320) is configured to store multiple drug titration algorithms; and
said drug titration module (130, 230, 330) is configured to select said drug titration algorithm among said multiple drug titration algorithms based on said algorithm selection signal.

6. The patient monitoring device according to any of the claims 1 to 5, wherein
said memory (220, 320) is configured to store said information of said current dose; and
said patient monitoring device (200, 300) further comprises:
a user input (260, 360) configured to generate an interaction signal representative of an interaction type selected by said patient by activating said user input (260, 360); and
a patient interaction module (270, 370) comprising:
a dose processor (272) configured to process said information of said current dose to generate information representing current dose over time for said patient; and
a characteristic processor (274) configured to process said current value to generate information representing current value of said medical characteristic over time for said patient, wherein said patient interaction module (270, 370) is configured to present, based on said interaction signal, one of said information generated by said dose processor (272) and said information generated by said characteristic processor (274) on said screen (250, 350).

7. The patient monitoring device according to claim 6, wherein
said patient interaction module (270, 370) comprises a symptoms processor (276) configured to present information urging said patient to enter a current symptom of said patient;
said user input (260, 360) is configured to generate, based on activation of said user input (260, 360), a symptoms signal representative of said current symptom;
said symptoms processor (276) is configured to store said symptoms signal in said memory (220, 320) and generate a hospitilization signal based on said symptoms signal, said current value and information of a previous hospitilization period of said patient stored in said memory (220, 320); and
said screen (250, 350) is configured to present, to said patient and based on said hospitalization signal, a warning of a risk for hospitalization.

8. The patient monitoring device according to any of the claims 1 to 7, wherein
said communication unit (110, 210, 310) is configured to receive, from an external weight scale (400), a signal representative of a current value of a weight of said patient; and
said drug titration module (130, 230, 330) is configured to process said current value and information of a starting dose, stored in said memory (120, 220, 320), according to said drug titration algorithm to output said information of said current dose.

9. A heart failure patient monitoring system (1) comprising:
a weight scale (400) comprising:
a processor (420) configured to generate a signal representative of a current weight of a patient standing on said weight scale (400); and
a communication unit (410) configured to transmit said signal;
a screen (150, 250, 350) configured to present, to said patient, information of multiple predefined education classes, wherein each education class of said multiple predefined education classes comprises at least one education instruction; and
a patient monitoring device (100, 200, 300) according to any of the claims 1 to 8, wherein said drug titration module (130, 230, 330) is configured to process said value according to a drug titration algorithm, stored in said memory (120, 220, 320), to output information of a current dose of a prescribed diuretic drug.
